# EUROPEAN PATENT APPLICATION

(11) **EP 1 262 165 A2**
(43) Date of publication of application: **04.12.2002**
(21) Application number: 02077093.9
(22) Date of filing: 28.05.2002
(51) Int. Cl.: A61K 7/06

(54) **Applying stain-free oil, apparatus and uses**

(30) Priority: 30.05.2001 GB 0113048
(71) Applicant: Shopville Limited, London W1F 9DP (GB)
(72) Inventor: Galvin, Daniel Joseph, Jr., Londonn W1U 7ES (GB)
(74) Representative: Buckley, Guy Julian

(57) **Abstract**

A method of and apparatus for applying a coating of stain-free oil to hair involving the use of a roller-ball applicator. Also disclosed are the use of a roller-ball applicator in a method of applying a coating of stain-free oil to hair and the use of a roller-ball applicator as a substitute for a brush and bowl in the application of a stain-free oil to hair. The stain-free oil is preferably a vegetable oil.

## Description

This invention is concerned with applying a coating of stain-free oil to hair. More particularly, though not exclusively, this invention is concerned with a method of applying a coating of stain-free oil to hair on a selected part of the scalp, such as on or near that part of the scalp known as the "hairline", and apparatus for performing said method.

Stain-free oils are used extensively in hair-colouring to prevent inadvertent colouring of hair on or near the hairline, which colouring may otherwise take place as a result of poor application of hair dye or bleach on hair in another part of the scalp.

Typically, stain-free oils are applied to the hairline by the use of a brush, which brush has previously been coated in oil by dipping the brush into the oil contained in a bowl. The oil is brushed onto the hair and scalp to form a coating of oil, which coating is of sufficient thickness so as to prevent any dye or bleach which may later be inadvertently splashed or spilt onto the hair from penetrating the oil coating and colouring the hair. There are a number of problems, however, associated with this age-old technique:
In some hair-dressing salons, particularly those which practice to high levels of hygiene, there can be a high wastage of oil, as unused oil retained in the bowl after sufficient oil has been applied to the scalp is thrown away after a single use. In other salons, where the oil is not disposed of after as single use, the oil on the brush or in the bowl often becomes contaminated with the shavings or clippings of cut hair, dyes and other salon contaminants, such as food particles, which can look offensive to a keen-eyed client and may cause a hygiene problem. Irrespective of how often the oil is used, the brush and bowl will eventually require washing before the bowl is refreshed with clean oil.
Applying the oil evenly to the scalp with a brush can at times be difficult, particularly for inexperienced personnel and self-stylists who tend to over-oil the hairline resulting in drips of oil running down the face and into the eyes or on the clothes.

There is a need to provide a method of applying a stain-free oil to hair which does not suffer from one or more of the above problems.

In accordance with a first aspect of the present invention, there is provided a method of applying a coating of stain-free oil to hair, which method comprises delivering said oil to the contact surface of a ball of a roller-ball applicator, and then contacting and transferring the oil on said surface with the hair to be coated by rolling said surface and the oil thereon over the hair.

In accordance with a second aspect, the present invention also provides apparatus suitable for use in the preceding method, which apparatus comprises a roller-ball applicator, comprising a reservoir and a ball having a contact surface remote from the reservoir, wherein the reservoir contains the stain-free oil. Preferably, the apparatus further comprises a closure that is adapted to protect said contact surface from contaminants when the apparatus is not in use and that is adapted to enable said contact surface to be exposed when the apparatus is required for use. Preferably, the closure is a screw-on lid, a hinged lid or a pull-off/push-on cap.

In a third aspect, the present invention provides the use of a roller-ball applicator, comprising a ball and reservoir, wherein the reservoir contains a stain-free oil, for applying a coating of stain-free oil to hair.

In a fourth aspect, the present invention provides, in a method of applying a coating of stain-free oil to hair, the use of a roller-ball applicator as a substitute for a brush and bowl.

Preferably, the hair to be coated with stain-free oil is on the scalp, more preferably on or near the hairline.

Roller-ball applicators are well known for use in association with e.g. under-arm deodorants, cosmetics, insect repellants and topical creams. However, such applicators have not hitherto been used for applying a coating of stain-free oil to scalp hair. The use of a roller-ball applicator as a substitute for a brush and bowl contributes a number of advantages: it eliminates wastage of unused oil, as all unused oil is retained in the reservoir of the applicator; it enables the oil to be applied evenly, thereby substantially reducing or eliminating the application of excess oil which could drip down the face; substantially no hair shavings or clippings, dyes or other salon contaminants can penetrate into the oil reservoir, thereby preventing the bulk of the oil retained in the applicator-reservoir from becoming contaminated; contaminating shavings and clippings of hair, dyes, etc., if any, which may become suspended in the oil film on the surface of the ball of the applicator can be easily wiped from the surface of the ball between uses; no washing up is required; and, as most users are already familiar with handling a roller-ball applicator, skilled stylist, unskilled hair-dressing personnel and self-stylist can readily use the applicator to apply an even amount of oil to the hair and scalp without difficulty and without having to learn a new technique. Surprisingly, it has been found that a roller-ball applicator is a most suitable piece of apparatus for overcoming the problems recited above.

Generally, any stain-free oil that is suitable for application to hair by brush will also be suitable for application to hair by a roller-ball applicator. Preferably, the stain-free oil comprises one or more esters of fatty acids, more preferably one or more esters of fatty acids in admixture with glycerol. Preferably, the stain-free oil comprises vegetable oil. For example, the stain-free oil may be a mixture of walnut oil and glycerol, such as a 50:50 admixture of walnut oil and glycerol. Though some experimental trial and error may be required to find the optimal oil for a particular roller-ball design, a person skilled in the art will be readily able to determine whether the viscosity of the oil is too high, which might prevent the ball from freely rolling during application, or too low, which might lead to leakage caused by inadequate film formation on the ball surface. A suitable stain-free oil will generally have a consistency at room temperature similar to a vegetable cooking oil at room temperature.

The present invention is preferably used to apply a coating of stain-free oil to hair in a selected part of the scalp adjacent to another part of the scalp where the hair therein is to be coloured, such as on or near the hairline. Applying the oil prevents any inadvertent spills or splashes of hair-colour from colouring the hair in that part of the scalp.

The present invention shall now be further described by way of exemplification in several of its various aspects.

A roller-ball applicator, having a visual design typical of the roller-ball applicators normally employed for applying e.g. an under-arm deodorant, comprises a reservoir and a ball, having a contact surface remote from the reservoir. A screw-on lid is placed over the ball to protect the contact surface when the apparatus is not in use. The reservoir contains a stain-free oil. In this particular example, the stain-free oil is a 50:50 admixture of walnut oil and glycerol, which at room temperature has a consistency similar to a sunflower cooking oil, available from Tesco plc, England.

In use, a coating of the stain-free oil is applied to the hairline of a person awaiting receipt of a hair dye. The coating is applied by holding the roller-ball applicator with the contact surface touching the scalp at the hairline. The roller-ball applicator is then moved along the hairline keeping the contact surface touching the scalp. In doing so, stain-free oil is transferred from the reservoir to the surface of the ball. As the ball is rolled along the scalp, stain-free oil on the contact surface of the ball is transferred to the scalp.

After application, the contact surface of the ball can be wiped with a tissue to remove any stain-free oil that might have been in contact with the person. Then the screw-on lid is placed over the ball and secured in position, thereby preventing any extraneous hair shavings or clippings from contaminating the contact surface before the apparatus is used again.

## Claims

1. A method of applying a coating of stain-free oil to hair, which method comprises delivering said oil to the contact surface of a ball of a roller-ball applicator, and then contacting and transferring the oil on said surface of the ball with the hair to be coated by rolling said surface of the ball and the oil thereon over the hair.

2. A method as claimed in claim 1, wherein the hair coated with stain-free oil is on the scalp.

3. A method as claimed in claim 2, wherein the hair coated with stain-free oil is on or near to the hairline.

4. Apparatus suitable for use in the method claimed in any one of claims 1 to 3, wherein the apparatus comprises a roller-ball applicator comprising a reservoir and a ball having a contact surface remote from the reservoir; **characterized in that** the reservoir contains a stain-free-oil.

5. Apparatus as claimed in claim 4, wherein the apparatus further comprises a closure that is adapted to protect said contact surface from contaminants when the apparatus is not in use and that is adapted to enable said contact surface to be exposed when the apparatus is required for use.

6. Apparatus as claimed in claim 5, wherein the closure is a screw-on lid, a hinged lid or a pull-off/push-on cap.

7. The use of a roller-ball applicator, comprising a ball and reservoir, wherein the reservoir contains a stain-free oil, for applying a coating of said stain-free oil to hair.

8. The use of a roller-ball applicator as a substitute for a brush and bowl in a method of applying a coating of stain-free oil to hair.

9. The method claimed in any one of claims 1 to 3, or the apparatus claimed in any one of claims 4 to 6, or the uses claimed in either of claims 7 or 8, wherein the stain-free oil comprises an ester of a fatty acid.

10. The method claimed in any one of claims 1 to 3, or the apparatus claimed in any one of claims 4 to 6, or the uses claimed in either of claims 7 or 8, wherein the stain-free oil comprises an ester of a fatty acid and glycerol.

11. The method claimed in any one of claims 1 to 3, or the apparatus claimed in any one of claims 4 to 6, or the uses claimed in either of claims 7 or 8, wherein the stain-free oil comprises vegetable oil.

12. The method claimed in any one of claims 1 to 3, or the apparatus claimed in any one of claims 4 to 6, or the uses claimed in either of claims 7 or 8, wherein the stain-free oil comprises a mixture of walnut oil and glycerol.
